# EUROPEAN PATENT SPECIFICATION

(11) **EP 3 355 178 B1**
(45) Date of publication and mention of the grant of the patent: **24.05.2023**
(21) Application number: 18153290.4
(22) Date of filing: 24.01.2018
(51) Int. Cl.: G06F 3/0488, A61B 8/00, G06F 3/042

(54) **ULTRASOUND DIAGNOSIS APPARATUS**
ULTRASCHALLDIAGNOSEVORRICHTUNG
APPAREIL DE DIAGNOSTIC À ULTRASONS

(30) Priority: 25.01.2017 JP 2017010847; 25.12.2017 JP 2017247135
(43) Date of publication of application: 01.08.2018
(73) Proprietor: Canon Medical Systems Corporation, Tochigi 324-8550 (JP)
(72) Inventor: SUGIO, Takeshi, Tochigi (JP); NABATAME, Tomio, Tochigi (JP); MASUDA, Takashi, Tochigi (JP); OZAWA, Asuka, Tochigi (JP)
(74) Representative: Marks & Clerk LLP

(56) References cited:
- EP-A1- 3 114 999
- EP-A2- 0 949 578
- WO-A1-2013/135299
- WO-A2-2013/148730
- US-A1- 2004 085 300
- US-A1- 2008 163 131
- US-A1- 2014 160 035

## Description

### FIELD

Embodiments described herein relate generally to an ultrasound diagnosis apparatus.

### BACKGROUND

Some conventional ultrasound diagnosis apparatuses receive operation instructions from an operator via physical operation switches such as button switches and dials and a touch command screen (TCS). The touch command screen includes a touch panel, and is an operation device that displays soft keys on the touch panel and receives an operation instruction corresponding to a soft key at a position where an operator's finger or the like has touched. Hereinafter, the touch command screen and the touch panel may sometimes be equated for the sake of explanation.

There are also known ultrasound diagnosis apparatuses that receive operation instructions via a touch command screen without the aforementioned physical operation switches. Such an ultrasound diagnosis apparatus displays soft keys each corresponding to a function that has been implemented by an operation instruction provided via a physical operation switch on the touch panel. The layout of the soft keys displayed on the touch panel can be dynamically changed according to various operation modes of the ultrasound diagnosis apparatus and operation instructions from the operator.

However, when the operator provides an operation instruction via the touch panel, inconvenience may occur in the operation. As one example may be cited the case where the operator performs touch typing. When using physical operation switches, the operator can perform touch typing while feeling the unevenness of the operation switches by the sense of touch in his/her fingers. On the other hand, when using a flat touch panel, it is difficult to perform touch typing while figuring out the position of each soft key, and the operator may make a mistake in providing an operation instruction. For example, when the ultrasound diagnosis apparatus is used in mass screening, operation instructions are provided according to a roughly determined routine, and quickness and accuracy are required in performing as many examinations as possible in a short period of time. The operation input method using the touch panel sometimes makes the operator feel inconvenient in such cases.

EP 3,114,999 A1 (SAMSUNG MEDISON CO LTD) describes an apparatus for processing medical images. The apparatus includes a storage unit storing fingerprints that respectively correspond to a plurality of functions, a user input unit detecting a fingerprint of a user, and a controller performing a function corresponding to the detected fingerprint, from among the functions

US 2014/160035 A1 (SAUER DIETMAR MICHAEL ET AL) describes apparatus, computer-implemented methods, and systems for interacting with computing systems using finger-specific input. In one general embodiment, a finger-specific input device can identify and differentiate each finger of a user. For example, the finger-specific input device may be able to detect features of each individual finger, such as fingerprints or detectable finger attachments, and to determine a set of fingers using the input device by camera or other visual cue, among others. A user can use multiple fingers to interact with a computing system and generate commands based on the use of a particular finger, a particular combination of fingers, and particular gestures of specific finger or combination of finger movements. In some implementations, specific commands are assigned to corresponding fingers and/or finger-specific multi-touch gestures in a program environment. In some implementations, users can define specific commands for specific fingers and/or finger-specific multi-touch gestures.

US 2008/0163131 A1 discloses a user interface system comprising an operation section for receiving an instruction inputted by a user; a finger position detecting section for detecting a group of first coordinate values, each indicating a current position of a fingertip, of a user, which is placed on an operation surface of the operation section; a component storing section for storing component data representing a GUI component and an assignment section for uniquely assigning the component data stored in the component storing section to each of the first coordinate values detected by the finger position detecting section.

### SUMMARY

Aspects of the present invention are defined in the independent claim.

Some preferred features are defined in the dependent claims.

### BRIEF DESCRIPTION OF THE DRAWINGS

Fig. 1 is a block diagram illustrating a configuration of an ultrasound diagnosis apparatus according to a first embodiment;
Fig. 2 is a schematic diagram of association information according to the first embodiment;
Fig. 3 is a schematic diagram illustrating a relationship between a sensor and a touch panel according to the first embodiment;
Fig. 4 is a schematic diagram of the association information displayed on a display according to the first embodiment;
Fig. 5 is a flowchart illustrating the operation of the ultrasound diagnosis apparatus according to the first embodiment;
Fig. 6 is a schematic diagram illustrating a relationship between association information and partial areas according to a first modification;
Fig. 7 is a block diagram illustrating a configuration of a second modification;
Fig. 8 is a block diagram illustrating a configuration of an ultrasound diagnosis apparatus according to a third modification;
Fig. 9 is a schematic diagram of a plurality of fingers touching on a touch panel according to the third modification;
Fig. 10 is a block diagram illustrating a configuration of an ultrasound diagnosis apparatus according to a fourth modification; and
Fig. 11 is a schematic diagram illustrating a relationship between a projector and a sensor and an operation area according to the fourth modification.

### DETAILED DESCRIPTION

In general, according to one embodiment, an ultrasound diagnosis apparatus is capable of receiving an operation instruction via a touch panel. The ultrasound diagnosis apparatus includes a storage, an identification unit, and a controller. The storage stores association information in which operation instructions are each associated with a finger type. The identification unit identifies the finger type of a finger that has touched any position on the touch panel. The controller performs one of the operation instructions associated with the finger type identified based on the association information.

Referring now to the drawings, a description is given of an ultrasound diagnosis apparatus according to embodiments.

### <First Embodiment>

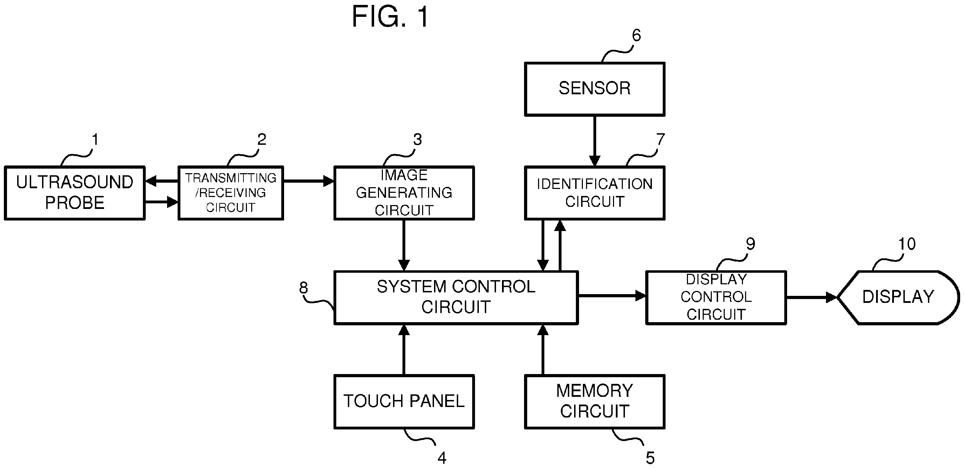

Fig. 1 is a block diagram illustrating a configuration of an ultrasound diagnosis apparatus according to a first embodiment. The ultrasound diagnosis apparatus of the first embodiment includes an ultrasound probe 1, a transmitting/receiving circuit 2, an image generating circuit 3, a touch panel 4, a memory circuit 5, a sensor 6, an identification circuit 7, a system control circuit 8, a display control circuit 9, and a display 10.

The ultrasound probe 1 transmits ultrasound waves to a subject and receives reflected waves therefrom. The ultrasound probe 1 outputs an echo signal based on the reflected waves from the subject to the transmitting/receiving circuit 2.

The transmitting/receiving circuit 2 supplies an electric signal to the ultrasound probe 1 according to a control signal from the system control circuit 8 to cause it to transmit ultrasound waves that have been beamformed to a predetermined focal point (i.e., subjected to transmission beamforming). Further, the transmitting/receiving circuit 2 receives the echo signal from the ultrasound probe 1. The transmitting/receiving circuit 2 performs a delay process on the echo signal, thereby converting the analog echo signal into a received signal with phased addition. The transmitting/receiving circuit 2 outputs the received signal to the image generating circuit 3.

The image generating circuit 3 generates an ultrasound image based on the received signal from the transmitting/receiving circuit 2. For example, the image generating circuit 3 performs band-pass filtering on the received signal. Thereafter, the image generating circuit 3 detects the envelope of the output signal, and performs a compression process on the detected data by logarithmic conversion. The image generating circuit 3 converts the received signal subjected to the compression process (ultrasound raster data) into a coordinate system for display (scan conversion), thereby generating an ultrasound image. The ultrasound image is displayed on the display 10 by the system control circuit 8 and the display control circuit 9.

The touch panel 4 receives an operation by an operator such as a doctor or a technician, and outputs an operation instruction corresponding to the content of the operation to the system control circuit 8. For example, in a general operation state, the touch panel 4 displays soft keys and detects a touch of the operator's finger or the like. The touch panel 4 outputs an operation instruction corresponding to a soft key displayed at the touched position detected to the system control circuit 8. In another operation state, the touch panel 4 receives an operation instruction by touch typing (details will be described later).

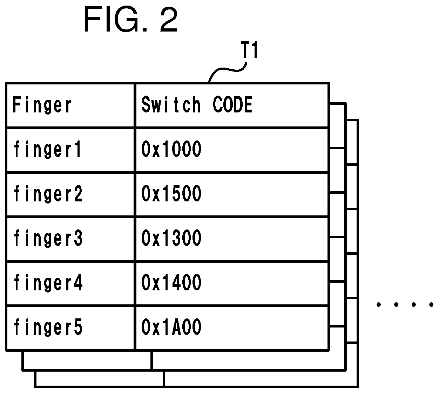

The memory circuit 5 is an example of the storage in the claims. The memory circuit 5 stores association information in which the type of a finger is associated with an operation instruction. Fig. 2 schematically illustrates the association information. For example, the association information T1 is table information. In the association information T1, the finger type (Finger: finger 1 to finger 5) and the operation instruction (Switch CODE: 0×1000, 0×1500, 0×1300, 0×1400, 0×1A00) are associated with each other. Specific types (thumb, index finger, ..., little finger) assigned to the finger type (each Finger) can be set and changed as appropriate by the operator or the like. In addition, specific operation instructions (Freeze, SET, NEXT, etc.) assigned to the operation instruction (each Switch CODE) can be set and changed as appropriate by the operator or the like.

Incidentally, the memory circuit 5 may store the association information in a plurality of tables. In this case, for example, the operator selects a desired one of the tables, and the association information of the selected table is used. Thereby, the operator can select a desired table from a plurality of tables having different associations between the finger type and the operation instruction.

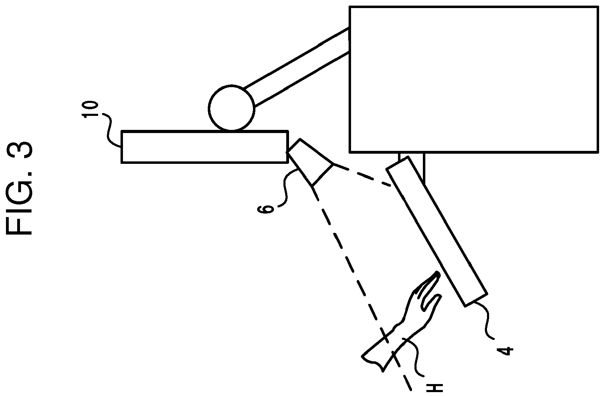

The sensor 6 detects the shape and size of the operator's hand. Example of the hardware used for the sensor 6 include an optical sensor, a camera, and the like. Fig. 3 is a schematic diagram illustrating the relationship between the sensor 6 and the touch panel 4. The sensor 6 is appropriately installed in a position where it can detect the hand H of the operator near the touch panel 4 (the detection range of the sensor 6 is schematically indicated by a broken line). For example, the sensor 6 detects the shape and size of the hand at every predetermined sampling rate, and outputs detection information indicating the shape and size detected to the identification circuit 7.

The identification circuit 7 is a processor that identifies the type of finger having touched the touch panel 4. For example, the identification circuit 7 stores in advance statistical information in which the shape and size of the hand are associated with the finger type. The identification circuit 7 compares the detection information from the sensor 6 with the statistical information, and thereby identifies the type of each finger of the hand detected by the sensor 6. In this manner, the identification circuit 7 can identify the type of each finger located around the touch panel 4. The identification circuit 7 performs this identification process at every predetermined sampling rate.

The system control circuit 8 is a processor that controls each part of the ultrasound diagnosis apparatus. For example, the system control circuit 8 stores a program for controlling each part of the ultrasound diagnosis apparatus and executes it. For example, the system control circuit 8 outputs an ultrasound image generated by the image generating circuit 3 to the display control circuit 9. The system control circuit 8 performs an operation instruction associated with the finger type identified by the identification circuit 7 based on the association information stored in the memory circuit 5. This operation instruction corresponds to an operation instruction provided by touch typing. Described below is the process performed in response to an operation instruction provided by touch typing.

The touch panel 4 in the operation state for receiving touch typing need not display soft keys. Having detected a touch of the operator's finger, the touch panel 4 outputs a touched position signal indicating the touched position on the touch panel 4 to the system control circuit 8 and the identification circuit 7. The identification circuit 7 compares the touched position signal with the type of the finger near the touch panel 4, and thereby identifies the type of the finger that has touched the touch panel 4. The system control circuit 8 compares the type of the finger that has touched the touch panel 4 with the association information stored in the memory circuit 5, and thereby specifies an operation instruction associated with the type of the finger that has touched the touch panel 4. When the memory circuit 5 stores the association information in a plurality of tables, the system control circuit 8 uses the association information of selected one of the tables. Then, the system control circuit 8 controls each part of the ultrasound diagnosis apparatus so as to perform the operation instruction specified.

The display control circuit 9 is a processor that displays ultrasound images and other information on the display 10. The display 10 is an example of the display in the claims. The display 10 is formed of a display device such as a liquid crystal display (LCD) or an organic electro-luminescence (EL) display. For example, the display control circuit 9 displays association information on the display 10.

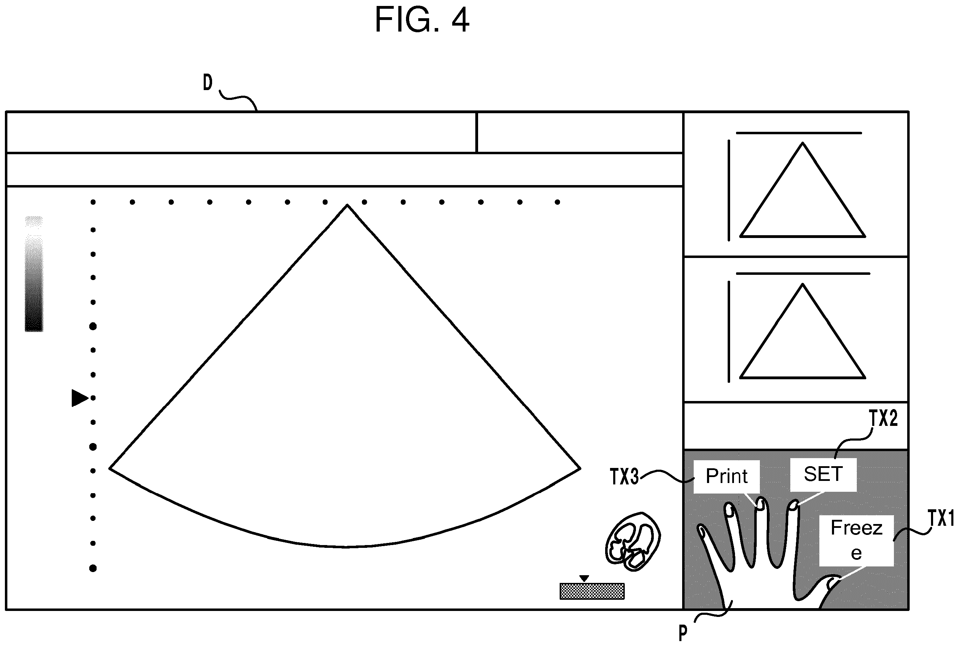

Fig. 4 is a schematic diagram of association information displayed on the display 10. For example, the display control circuit 9 displays association information alongside the ultrasound image. In the example of Fig. 4, a schematic illustration P of the hand and texts (TX1, TX2, TX3) each indicating an operation instruction associated with each finger are displayed at the lower right of the display screen D. The specific design for displaying the association information is not limited to such an illustration and text information, but may be set appropriately.

As described above, the operation instruction is associated with the finger type, and the operation instruction specified by the type of the finger having touched the touch panel 4 is performed. This facilitates operator's touch typing without looking at the touch panel 4. For example, in the case where an operation instruction "Freeze" is associated with the middle finger as a finger type, when the operator wishes to perform the Freeze operation during an ultrasound examination, he/she can perform the Freeze operation by touching the touch panel 4 with the middle finger. At this time, regardless of the position where the middle finger touches on the touch panel 4, the operation instruction of Freeze is entered. Thus, while viewing an ultrasound image displayed on the display 10, the operator can perform a simple operation of touching any position on the touch panel 4 with the middle finger without looking at the touch panel 4. When the operator wishes to enter another operation instruction, the operator may touch the touch panel 4 with the finger in association with the operation instruction.

The operation instructions set in the association information may include a switch instruction for switching on/off of the touch typing operation. For example, in the case where the switch instruction is associated with the thumb as a finger type, when the operator wishes to perform touch typing, he/she can turn on the touch typing operation by touching the touch panel 4 with the thumb. At this time, the touch typing operation can be switched on no matter where the thumb touches on the touch panel 4. Then, while touching the touch panel 4 with the thumb, the operator touches the touch panel 4 with another finger to enter an operation instruction.

While the finger associated with the switch instruction (the thumb in this example) is touching the touch panel 4, the identification circuit 7 identifies the type of another finger that has touched the touch panel 4 with the finger associated with the switch instruction. The system control circuit 8 compares the type of the other finger thus identified with the association information, and thereby specifies an operation instruction associated with the type of the other finger. Then, the system control circuit 8 controls each part of the ultrasound diagnosis apparatus so as to perform the operation instruction specified.

When the finger associated with the switch instruction (the thumb in this example) is not touching the touch panel 4, that is, when the identification circuit 7 does not identify the thumb, the display control circuit 9 displays soft keys each corresponding to an operation instruction on the touch panel 4. In this state, the touch typing operation is OFF. In this OFF state, the operator performs operation input by touching a soft key corresponding to a desired operation instruction in the same manner as the conventional soft key input.

As described above, with the configuration that the operator can operate while switching on/off the touch typing operation, the operator can perform operation input on the ultrasound diagnosis apparatus while suitably switching between the touch typing operation and the conventional soft key operation. Thereby, the operator can operate the touch panel while appropriately switching the operation input mode. Thus, the operability of the touch panel can be improved.

Besides, operation instructions provided by touch typing may include the display and movement of a cursor on the display 10. Examples of the cursor include various cursors used by common pointing devices, measurement calipers for measuring the dimension of tissue depicted in an ultrasound image, and the like. For example, as a result of finger type identification by the identification circuit 7, when the finger is the one associated with the movement operation, the display control circuit 9 moves the position of the cursor according to the movement of the finger. Thereby, while viewing an ultrasound image displayed on the display 10, the operator can move the cursor by moving the finger touching the touch panel 4 without looking at the touch panel 4.

Further, an operation instruction may be associated with a combination of the two or more types of fingers. For example, when the above-described cursor movement operation is associated with the index finger and the middle finger and both the index finger and the middle finger touch the touch panel 4, the display control circuit 9 moves the cursor according to the movement of these two fingers. In this case, the identification circuit 7 identifies the types of all fingers that are touching the touch panel 4 at the same time.

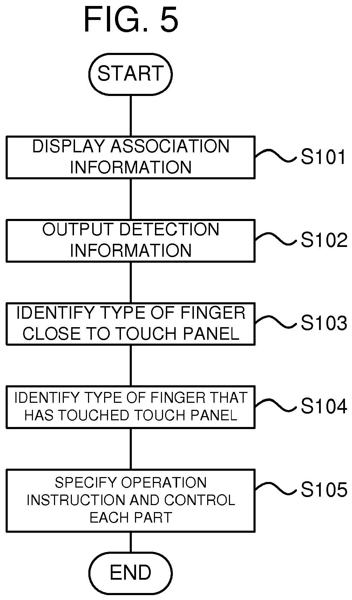

Fig. 5 is a flowchart illustrating the operation of the ultrasound diagnosis apparatus according to the first embodiment. In the following, an example will be described in which the operator performs touch typing while watching an ultrasound image during an ultrasound examination.

Step S101: When the touch typing operation is ON, the display control circuit 9 sequentially displays ultrasound images with association information in which the finger type and the operation instruction are associated with each other on the display 10.

Step S102: When the operator puts his/her hand close to the touch panel 4, the sensor 6 detects the shape and size of the hand of the operator. The sensor 6 outputs detection information indicating the shape and size detected to the identification circuit 7.

Step S103: The identification circuit 7 compares the detection information from the sensor 6 with statistical information in which the shape and size of the hand are associated with the finger type, and thereby identifies the type of each finger of the hand detected by the sensor 6.

Step S 104: The touch panel 4 outputs a touched position signal indicating the position where the finger touched to the system control circuit 8 and the identification circuit 7. The identification circuit 7 identifies the type of the finger that has touched the touch panel 4 by comparing the touched position signal with the type of the finger near the touch panel 4.

Step S 105: The system control circuit 8 compares the type of the finger that has touched the touch panel 4 with the association information stored in the memory circuit 5, and thereby specifies an operation instruction associated with the type of the finger that has touched the touch panel 4. Then, the system control circuit 8 controls each part of the ultrasound diagnosis apparatus so as to perform the operation instruction specified.

With the ultrasound diagnosis apparatus according to the first embodiment, the operator can perform easy and convenient touch typing by touching any position on the touch panel 4 with a finger corresponding to a desired operation instruction without looking at the touch panel 4. Thereby, even when a flat touch panel is used, it is possible to improve the quickness and accuracy of operation input using the touch panel.

### <First Modification>

An ultrasound diagnosis apparatus according to a first modification of the first embodiment will be described. In the following, differences from the ultrasound diagnosis apparatus of the first embodiment will be mainly described. The same description as already described in the first embodiment may not be repeated.

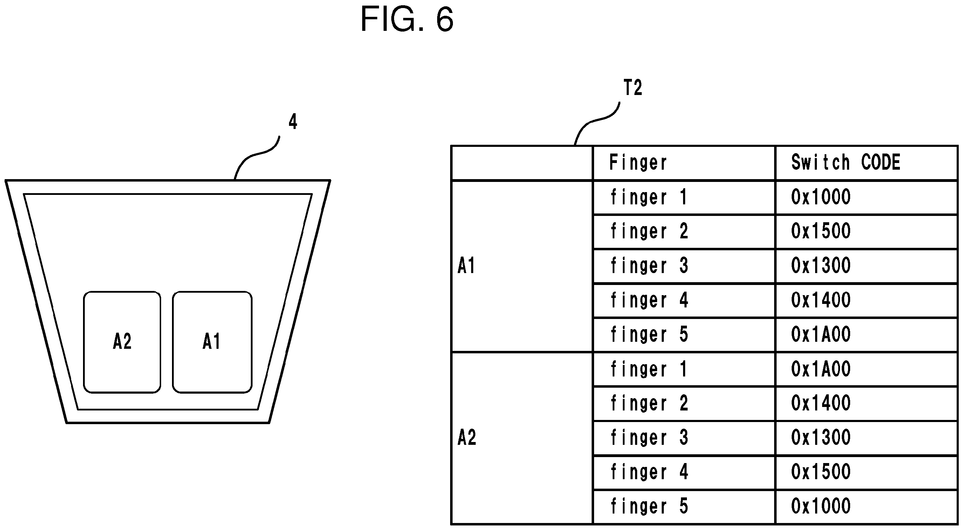

The memory circuit 5 of the first modification stores association information for each partial area of the touch panel 4. Fig. 6 is a schematic diagram illustrating the relationship between the partial area and the association information stored in the memory circuit 5 of the first modification. In the following, an example will be described in which a partial area A1 is set at the lower right of the touch panel 4 and the partial area A2 is set at the lower left of the touch panel 4. These partial areas (A1, A2) may be preset or may be set by operator's operation. In association information T2, the finger type (Finger: finger 1 to finger 5) and the operation instruction (Switch CODE: 0×1000, 0×1500, 0×1300, 0×1400, 0×1A00) are associated with each other with respect to each of the partial areas A1 and A2.

At the time of the touch typing operation, the system control circuit 8 specifies a partial area touched by the finger based on the touched position signal from the touch panel 4. The system control circuit 8 compares association information corresponding to the partial area with the type of the finger that has touched the touch panel 4 to thereby specify an operation instruction associated with the type of the finger that has touched the touch panel 4 in the association information. Then, the system control circuit 8 controls each part of the ultrasound diagnosis apparatus so as to perform the operation instruction specified.

Normally, when diagnosing with an ultrasound diagnosis apparatus, the operator holds an ultrasound probe on the right hand and performs operation input on the touch panel with the left hand. However, depending on the positional relationship between the ultrasound diagnosis apparatus and the bed, there are cases where the operator holds an ultrasound probe on the left hand and performs operation input on the touch panel with the right hand. With the ultrasound diagnosis apparatus of the first modification, the association information can be set in a partial area of the touch panel where the operator can easily touch according to the positional relationship. Thereby, it is possible to improve the operability for performing operation input according to the positional relationship between the ultrasound diagnosis apparatus and the bed.

### <Second Modification>

An ultrasound diagnosis apparatus according to a second modification of the first embodiment will be described, which does not form part of the claimed subject-matter. In the following, differences from the ultrasound diagnosis apparatus of the first embodiment will be mainly described. The same description as already described in the first embodiment may not be repeated.

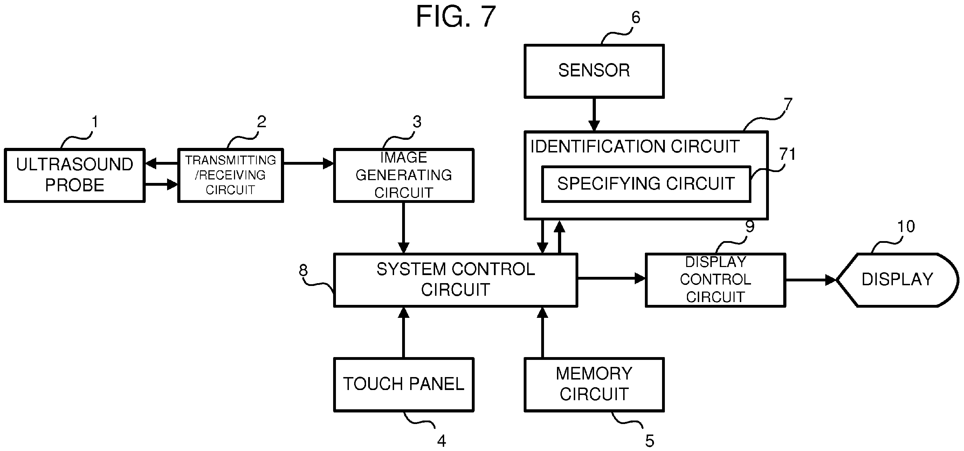

Fig. 7 is a block diagram illustrating a configuration of a second modification. The identification circuit 7 of the second modification includes a specifying circuit 71. The specifying circuit 71 is a processor that specifies the operator based on a detection result obtained by the sensor. For example, the specifying circuit 71 stores shape and size information indicating the shape and size of the hand with respect to each operator ID. The specifying circuit 71 compares detection information from the sensor 6 with the shape and size information, and thereby specifies the operator. The specifying circuit 71 outputs the operator ID of the operator specified to the system control circuit 8.

The memory circuit 5 stores association information with respect to each operator ID. For example, the operator can prepare association information according to the habit and preference of his/her operation and store it in the memory circuit 5 in advance. The system control circuit 8 performs an operation instruction based on the association information corresponding to the operator ID from the specifying circuit 71. For example, when the operator puts his/her hand close to the touch panel 4, the sensor 6 outputs detection information to the specifying circuit 71. The specifying circuit 71 specifies the operator based on the detection information, and outputs the operator ID of the operator specified to the system control circuit 8. The system control circuit 8 compares the type of the finger that has touched the touch panel 4 with the association information corresponding to the operator ID, and thereby specifies an operation instruction associated with the type of the finger that has touched the touch panel 4. Then, the system control circuit 8 controls each part of the ultrasound diagnosis apparatus so as to perform the operation instruction specified.

With the ultrasound diagnosis apparatus of the second modification, association information is automatically selected according to the preference of each operator. Thereby, the operability of the touch panel for operation input can be improved according to the preference of each operator.

### <Third Modification>

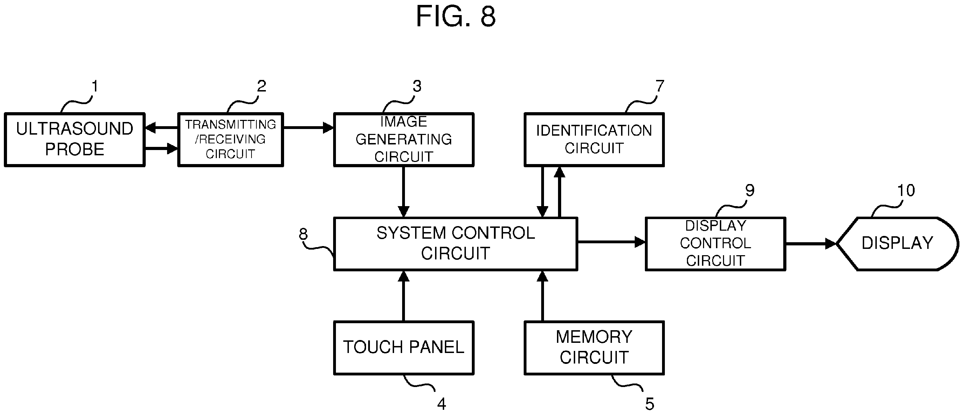

Fig. 8 is a block diagram illustrating a configuration of an ultrasound diagnosis apparatus according to a third modification. Differently from the first embodiment, the ultrasound diagnosis apparatus of the third modification identifies the type of the finger without using the sensor 6. In the following, differences from the ultrasound diagnosis apparatus of the first embodiment will be mainly described. The same description as already described in the first embodiment may not be repeated.

When the operator touches the touch panel 4 with a plurality of fingers, the touch panel 4 detects a position touched by each of the fingers and outputs touched position information to the system control circuit 8 and the identification circuit 7. Thereby, the identification circuit 7 detects the positions of the fingers that are touching the touch panel 4.

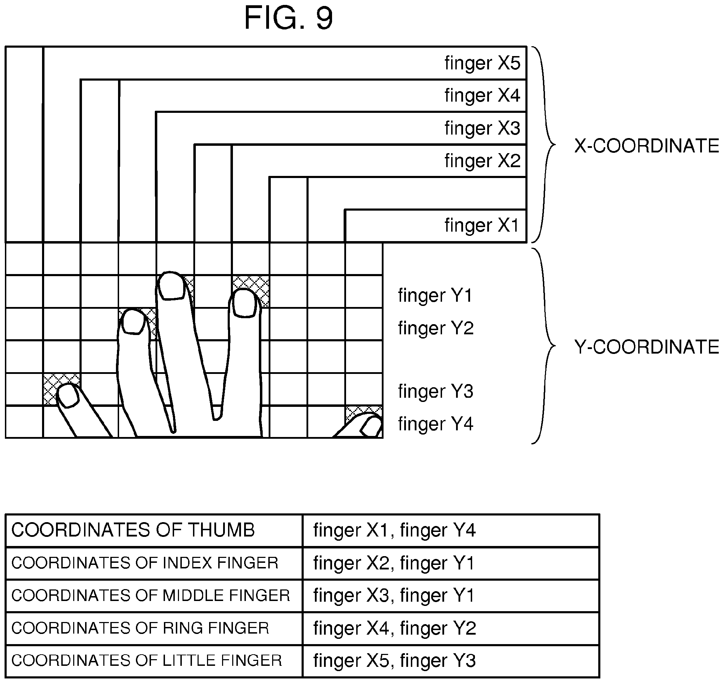

The identification circuit 7 identifies the type of each of the fingers based on the positional relationship of the fingers detected. Fig. 9 is a schematic diagram of a plurality of fingers touching the touch panel 4. The identification circuit 7 stores in advance statistical information on the shape of the hand. The statistical information includes the ratio of the lengths of the fingers and the like. With this, the relative positional relationship when each finger touches the touch panel 4 can be obtained. The identification circuit 7 compares the statistical information with the touched position information on the touch panel 4, and thereby individually identifies the type of each finger that has touched each touched position. In Fig. 9, the touched position is indicated by coordinates (finger X1 to finger X5, finger Y1 to finger Y4) with respect to each finger type identified.

Among the fingers touching the touch panel 4, the operator can once release a finger corresponding to a desired operation instruction from the touch panel 4. At this time, the other fingers are still touching the touch panel 4. Then, the operator can enter a desired operation instruction by touching again the touch panel 4 with the finger corresponding to the desired operation instruction.

At this time, the identification circuit 7 compares touched position information indicating a position touched by the finger that has touched again the touch panel 4 with the coordinates of the fingers identified before the finger has touched again the touch panel 4, and identifies the type of the finger that has touched again the touch panel 4. For example, in the example of Fig. 9, when the touched position information of the finger that has touched again the touch panel 4 indicates the coordinates of "finger X3, finger Y1", the identification circuit 7 identifies the type of the finger that has touched again as "middle finger". Incidentally, when a finger touches again the touch panel 4, the coordinates may differ from the previous coordinates. The allowable range of difference used for the identification may be appropriately set. Then, the system control circuit 8 controls each part of the ultrasound diagnosis apparatus so as to perform an operation instruction associated with the type of the finger identified.

Also with the ultrasound diagnosis apparatus of the third modification, the operator can perform easy and convenient touch typing by touching any position on the touch panel 4 with a finger corresponding to a desired operation instruction without looking at the touch panel 4. Thereby, even when a flat touch panel is used, it is possible to improve the quickness and accuracy of operation input using the touch panel.

### <Fourth Modification>

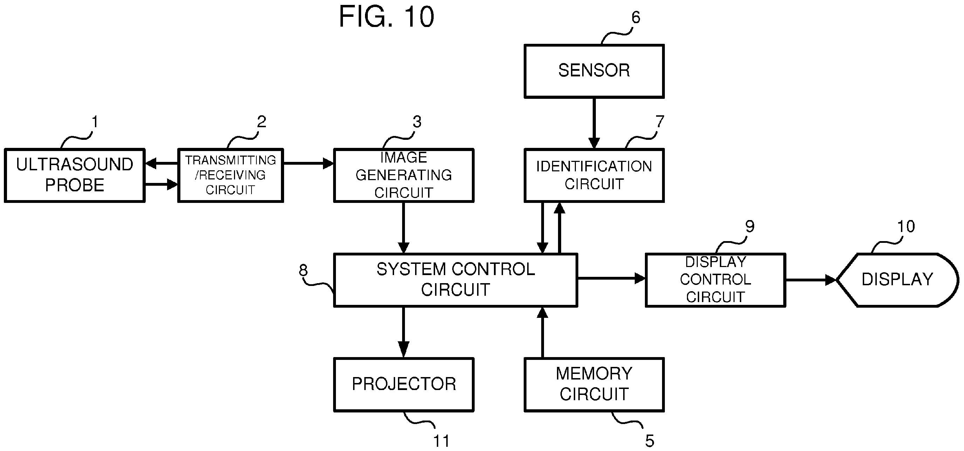

Fig. 10 is a block diagram illustrating a configuration of an ultrasound diagnosis apparatus according to a fourth modification, which does not form part of the claimed subject-matter. Differently from the first embodiment, the ultrasound diagnosis apparatus of the fourth modification is configured to be capable of receiving operation instructions without using a touch panel. In the following, differences from the first embodiment will be mainly described. The same description as already described in the first embodiment may not be repeated.

A projector 11 projects light indicating a predetermined operation area. The projected light may be visible light or non-visible light. The light projected by the projector 11 is only required to indicate at least the outer edge of the operation area. A general light source, an optical modulator, a lens, and the like may be appropriately used for the hardware configuration of the projector 11.

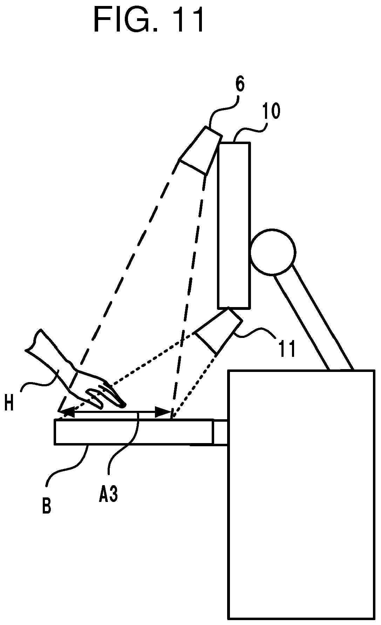

The sensor 6 detects the shape and size of the operator's hand in the vicinity of the operation area. Fig. 11 is a schematic diagram illustrating the relationship between the projector 11 and the sensor 6 and the operation area A3. The projector 11 projects light that indicates the operation area A3 onto a nearby flat plate or a bed. The function of the operation area A3 thus indicated corresponds to the function of the operation surface of the touch panel 4 in the above embodiment. The sensor 6 is appropriately installed in a position where it can detect the hand H of the operator in the vicinity of the operation area A3. For example, the detection range of the sensor 6 is determined so as to detect the hand H located within a predetermined distance from the flat plate B and in the operation area A3. Specific numerical values of the detection range may be determined as appropriate. For example, the sensor 6 detects the shape and size of the hand H of the operator at each predetermined sampling rate, and outputs detection information indicating the shape and size detected to the identification circuit 7. The identification circuit 7 identifies a finger in the vicinity of the operation area A3 as in the above embodiment.

With the ultrasound diagnosis apparatus according to the fourth modification, operation instructions can be provided without using the touch panel 4 by projecting an operation area on a flat plate, a desk, a wall, a bed, or the like around the ultrasound diagnosis apparatus and using the operation area as the operation surface of the touch panel 4. Thus, it is possible to perform touch typing input using the flat operation area, and the quickness and accuracy of operation input can be improved.

The term "processor" as used herein refers to a circuit such as, for example, a central processing unit (CPU), a graphics processing unit (GPU), an application specific integrated circuit (ASIC), a programmable logic device including a simple programmable logic device (SPLD) and a complex programmable logic device (CPLD), a field programmable gate array (FPGA), or the like. The processor reads programs out of a memory circuit and executes them to thereby realize the functions. The programs need not necessarily be stored in a memory circuit, but may be directly incorporated in the circuit of the processor. In this case, the processor realizes the functions by reading and executing the programs incorporated in the circuit. Each processor of the embodiments need not necessarily be configured as a single circuit. A plurality of independent circuits may be combined to form a single processor for implementing the functions. Besides, a plurality of constituent elements in Fig. 1 may be integrated into one processor to realize the functions.

According to the ultrasound diagnosis apparatus of at least one embodiment described above, it is possible to improve the operability of the touch panel for operation input.

While certain embodiments have been described, these embodiments have been presented by way of example only, and are not intended to limit the scope of the invention, which is limited solely by the appended claims.

## Claims

1. An ultrasound diagnosis apparatus capable of receiving an operation instruction via a touch panel, the apparatus comprising:
a storage (5) configured to store association information (T1, T2) in which operation instructions are each associated with a finger type; and
processing circuitry configured to
identify finger type of a finger that has touched any position on the touch panel (4), and
perform one of the operation instructions associated with the finger type identified based on the association information (T1, T2), wherein
the processing circuitry is further configured to
detect, by the touch panel, positions of a plurality of fingers of a hand that are touching the touch panel (4),
identify finger type of each of the fingers based on a comparison of the detected positions of the fingers with stored statistical information on the shape of the hand, and
compare position of one of the fingers that is released from the touch panel (4) and then touches the touch panel (4) again with the detected positions of the plurality of fingers to identify finger type of the finger,
wherein the processing circuitry is further configured to display the association information (T1, T2) on a display (10).

2. The ultrasound diagnosis apparatus of claim 1, wherein
the operation instructions include a switch instruction for turning on and off touch typing operation, and
the processing circuitry is further configured to perform one of the operation instructions associated with the finger type identified based on the association information (T1, T2) when the touch typing operation is turned on.

3. The ultrasound diagnosis apparatus of claim 1, wherein
in the association information (T1, T2), a switch instruction for switching control state of the processing circuitry is associated with a predetermined finger type as one of the operation instruction, and
the processing circuitry is further configured to
identify finger types of a plurality of fingers that have touched the touch panel (4), and
when the finger types identified include the predetermined finger type, perform an operation instruction associated with another of the finger types identified.

4. The ultrasound diagnosis apparatus of claim 3, wherein the processing circuitry is further configured to display soft keys according to the operation instructions on the touch panel (4) when the finger types identified do not include the predetermined finger type.

5. The ultrasound diagnosis apparatus of claim 1, wherein
the processing circuitry is further configured to display a cursor on a display (10),
the operation instructions include a move instruction to move the cursor, and
when the finger type identified is associated with the move instruction, the processing circuitry move display position of the cursor according to movement of the finger.

6. The ultrasound diagnosis apparatus of claim 1, wherein
the storage (5) is further configured to store the association information (T1, T2) with respect to each partial area (A1, A2) of the touch panel (4), and
the processing circuitry is further configured to identify finger type of a finger that has touched the partial area (A1, A2).

7. The ultrasound diagnosis apparatus of claim 1, further comprising a sensor (6) configured to detect shape and size of a hand of an operator, wherein
the processing circuitry is further configured to specify the operator based on a detection result obtained by the sensor (6),
the storage (5) is further configured to store the association information (T1, T2) with respect to each operator ID, and
the processing circuitry is further configured to perform the operation instructions based on the association information(T1, T2) corresponding to the operator ID of the operator specified.

## Patentansprüche

1. Ultraschall-Diagnosegerät, das dazu in der Lage ist, eine Betriebsanweisung über ein Berührungsfeld zu empfangen, wobei das Gerät Folgendes umfasst:
einen Speicher (5), der dafür konfiguriert ist, Verknüpfungsinformationen (T1, T2) zu speichern, in denen Betriebsanweisungen jeweils mit einer Fingerart verknüpft sind, und
Verarbeitungsschaltungen, die dafür konfiguriert sind,
die Fingerart eines Fingers zu identifizieren, der eine beliebige Position auf dem Berührungsfeld (4) berührt hat, und
eine der Betriebsanweisungen auszuführen, die mit der auf Grundlage der Zuordnungsinformationen (T1, T2) identifizierten Fingerart verknüpft sind, wobei
die Verarbeitungsschaltungen ferner dafür konfiguriert sind,
durch das Berührungsfeld Positionen einer Vielzahl von Fingern einer Hand zu erkennen, die das Berührungsfeld (4) berühren,
die Fingerart jedes der Finger auf Grundlage eines Vergleichs der erkannten Positionen der Finger mit gespeicherten statistischen Informationen über die Form der Hand zu identifizieren, und
die Position eines der Finger, der von dem Berührungsfeld (4) gelöst wird und danach das Berührungsfeld (4) erneut berührt, mit den erkannten Positionen der Vielzahl von Fingern zu vergleichen, um die Fingerart des Fingers zu identifizieren,
wobei die Verarbeitungsschaltungen ferner dafür konfiguriert sind, die Zuordnungsinformationen (T1, T2) auf einer Anzeige (10) anzuzeigen.

2. Ultraschall-Diagnosegerät nach Anspruch 1, wobei
die Betriebsanweisungen eine Schaltanweisung zum Ein- und Ausschalten eines Berührungstippbetriebs einschließen, und
die Verarbeitungsschaltungen ferner dafür konfiguriert sind, eine der Betriebsanweisungen auszuführen, die mit der auf Grundlage der Zuordnungsinformationen (T1, T2) identifizierten Fingerart verknüpft sind, wenn der Berührungstippbetrieb eingeschaltet ist.

3. Ultraschall-Diagnosegerät nach Anspruch 1, wobei
in den Zuordnungsinformationen (T1, T2) eine Schaltanweisung zum Schalten eines Steuerungszustandes der Verarbeitungsschaltungen mit einer vorbestimmten Fingerart als eine der Betriebsanweisungen verknüpft ist, und
die Verarbeitungsschaltungen ferner dafür konfiguriert sind,
Fingerarten einer Vielzahl von Fingern zu identifizieren, die das Berührungsfeld (4) berührt haben, und,
wenn die identifizierten Fingerarten die vorbestimmte Fingerart einschließen, eine Betriebsanweisung auszuführen, die mit einer anderen der identifizierten Fingerarten verknüpft ist.

4. Ultraschall-Diagnosegerät nach Anspruch 3, wobei die Verarbeitungsschaltungen ferner dafür konfiguriert sind, Softkeys entsprechend den Betriebsanweisungen auf dem Berührungsfeld (4) anzuzeigen, wenn die identifizierten Fingerarten die vorbestimmte Fingerart nicht einschließen.

5. Ultraschall-Diagnosegerät nach Anspruch 1, wobei
die Verarbeitungsschaltungen ferner dafür konfiguriert sind, einen Eingabezeiger auf einer Anzeige (10) anzuzeigen,
die Betriebsanweisungen eine Bewegungsanweisung einschließen, um den Eingabezeiger zu bewegen, und
wenn die identifizierte Fingerart mit der Bewegungsanweisung verknüpft ist, die Verarbeitungsschaltungen die Anzeigeposition des Eingabezeigers entsprechend einer Bewegung des Fingers bewegen.

6. Ultraschall-Diagnosegerät nach Anspruch 1, wobei
der Speicher (5) ferner dafür konfiguriert ist, die Zuordnungsinformationen (T1, T2) in Bezug auf jeden Teilbereich (A1, A2) des Berührungsfeldes (4) zu speichern, und
die Verarbeitungsschaltungen ferner dafür konfiguriert sind, die Fingerart eines Fingers zu identifizieren, der den Teilbereich (A1, A2) berührt hat.

7. Ultraschall-Diagnosegerät nach Anspruch 1, das ferner einen Sensor (6) umfasst, der dafür konfiguriert ist, Form und Größe einer Hand eines Bedieners zu erkennen, wobei
die Verarbeitungsschaltungen ferner dafür konfiguriert sind, den Bediener auf Grundlage eines durch den Sensor (6) erhaltenen Erkennungsergebnisses zu bestimmen,
der Speicher (5) ferner dafür konfiguriert ist, die Zuordnungsinformationen (T1, T2) in Bezug auf jede Bediener-ID zu speichern, und
die Verarbeitungsschaltungen ferner dafür konfiguriert sind, die Betriebsanweisungen auf Grundlage der Zuordnungsinformationen (T1, T2) auszuführen, die der Bediener-ID des bestimmten Bedieners entsprechen.

## Revendications

1. Appareil de diagnostic à ultrasons capable de recevoir une instruction de fonctionnement via un panneau tactile, l'appareil comprenant :
une mémoire (5) configurée pour stocker des informations d'association (T1, T2) dans lesquelles les informations de fonctionnement sont chacune associées à un type de doigt ; et
une circuiterie de traitement configurée pour :
identifier le type de doigt d'un doigt qui a touché une quelconque position sur le panneau tactile (4) ; et
exécuter l'une des instructions de fonctionnement associées au type de doigt identifié sur la base des informations d'association (T1, T2) ; dans lequel :
la circuiterie de traitement est en outre configurée pour :
détecter, par le panneau tactile, des positions d'une pluralité de doigts d'une main qui touchent le panneau tactile (4) ;
identifier le type de doigt de chacun des doigts sur la base d'une comparaison des positions détectées des doigts avec des informations statistiques stockées sur la forme de la main ; et
comparer la position de l'un des doigts qui s'est libéré du panneau tactile (4) et touche ensuite à nouveau le panneau tactile (4) avec les positions détectées de la pluralité de doigts pour identifier le type de doigt du doigt ;
dans lequel la circuiterie de traitement est en outre configurée pour afficher les informations d'association (T1, T2) sur un affichage (10).

2. Appareil de diagnostic à ultrasons selon la revendication 1, dans lequel
les instructions de fonctionnement incluent une instruction de commutation pour activer et désactiver une opération de frappe tactile ; et
la circuiterie de traitement est en outre configurée pour exécuter une des instructions de fonctionnement associées au type de doigt identifié sur la base des informations d'association (T1, T2) lorsque l'opération de frappe tactile est activée.

3. Appareil de diagnostic à ultrasons selon la revendication 1, dans lequel
dans les informations d'association (T1, T2), une instruction de commutation pour commuter un état de commande de la circuiterie de traitement est associée à un type de doigt prédéterminé comme l'une des instructions de fonctionnement ; et
la circuiterie de traitement est en outre configurée pour :
identifier les types de doigts d'une pluralité de doigts qui ont touché le panneau tactile (4) ; et
lorsque les types de doigts identifiés incluent le type de doigt prédéterminé, exécuter une instruction de fonctionnement associée à un autre des types de doigts identifiés.

4. Appareil de diagnostic à ultrasons selon la revendication 3, dans lequel la circuiterie de traitement est en outre configurée pour afficher des touches programmables en fonction des instructions de fonctionnement sur le panneau tactile (4) lorsque les types de doigts identifiés n'incluent pas le type de doigt prédéterminé.

5. Appareil de diagnostic à ultrasons selon la revendication 1, dans lequel
la circuiterie de traitement est en outre configurée pour afficher un curseur sur un affichage (10) ;
les instructions de fonctionnement incluent une instruction de déplacement pour déplacer le curseur ; et
lorsque le type de doigt identifié est associé à l'instruction de déplacement, la circuiterie de traitement déplace la position d'affichage du curseur en fonction d'un déplacement du doigt.

6. Appareil de diagnostic à ultrasons selon la revendication 1, dans lequel
la mémoire (5) est en outre configurée pour stocker les informations d'association (T1, T2) par rapport à chaque zone partielle (A1, A2) du panneau tactile (4) ; et
la circuiterie de traitement est en outre configurée pour identifier le type de doigt d'un doigt qui a touché la zone partielle (A1, A2).

7. Appareil de diagnostic à ultrasons selon la revendication 1, comprenant en outre un capteur (6) configuré pour détecter la forme et la taille d'une main d'un opérateur, dans lequel
la circuiterie de traitement est en outre configurée pour spécifier l'opérateur sur la base d'un résultat de détection obtenu par le capteur (6) ;
la mémoire (5) est en outre configurée pour stocker les informations d'association (T1, T2) par rapport à chaque identifiant d'opérateur ; et
la circuiterie de traitement est en outre configurée pour exécuter les instructions de fonctionnement sur la base des informations d'association (T1, T2) correspondant à l'identifiant d'opérateur de l'opérateur spécifié.
